# EUROPEAN PATENT SPECIFICATION

(11) **EP 3 787 742 B1**
(45) Date of publication and mention of the grant of the patent: **24.08.2022**
(21) Application number: 19733102.8
(22) Date of filing: 02.05.2019
(51) Int. Cl.: A61N 5/06, H01S 3/00

(54) **IMPROVED LOW POWER LASER THERAPY DEVICE**
VERBESSERTE LASERVORRICHTUNG MIT NIEDRIGEM STROMVERBRAUCH
DISPOSITIF LASER DE THÉRAPIE À FAIBLE PUISSANCE AMÉLIORÉ

(30) Priority: 03.05.2018 HU 1800145
(43) Date of publication of application: 10.03.2021
(73) Proprietor: Rózsa, Tamás, 2146 Mogyoród (HU)
(72) Inventor: Rózsa, Tamás, 2146 Mogyoród (HU)
(74) Representative: Danubia Patent & Law Office LLC
(86) International application number: PCT/HU2019/050018
(87) International publication number: WO 2019/211638

(56) References cited:
- WO-A1-2017/001876
- US-A1- 2006 047 330
- US-A1- 2008 125 835
- US-A1- 2009 216 299
- US-A1- 2014 005 756

## Description

The invention relates to an improved low power therapy laser device that comprises a housing, a laser light source arranged in the housing, a beam expander arranged in the beam path of the laser and also diverting lenses built together or separately from the beam expander.

The biostimulating effect of low power lasers are widely known. In 1970 professor Mester, Endre has discovered that wounds that do not heal or where the healing is slow will heal under the effect of irradiation with laser light of low intensity (called also as soft lasers). The phenomenon was called as biostimulation. These lasers emit single colour, coherent, polarized and parallel light beams. The effect of biostimulation is due to the small bandwidth of wavelengths (colour), coherence and polarization of the light. The summary of the related research can be found in the paper of Mester, E, and Mester, A, entitled as "The biomedical effects of laser applications" (Lasers Surg. Med 5, 1985, pp 31-39).

The pioneer work of professor Mester was followed by numerous other research, and the relevant literature is substantial, it has reached almost a size that cannot be overlooked at all. One of the most detailed and broadest summaries can be found in the book of Lars Hode & Jan Tuner "Laser Phototherapy - Clinical Practice and Scientific Background" which is 900 pages long and has 2500 reference ( Prima Books edition, Coeymans Hollow, NY, USA - extended edition 2015). In this publication the main attention was directed to therapeutic applications of small power laser light.

The biostimulation effects of light (not limited only to laser applications) is broadly described by the study of professor Michael R. Hamblin (Department of Dermatology, Harvard Medical School, BAR 414, Wellman Center for Photomedicine, Massachutes General Hospital: Mechanisms of Low Level Light Therapy, published on August 14, 2008 which can be read e.g. at the web address: hhtp://www.photobiology.info/Hamblin.html. Chapter 7 of the study deals with the issues yet to be solved, and it is described there what properties of light are responsible for the biostimulation effect, namely the spectral distribution, the monochromatic nature, the degree of polarization, the specific light intensity or the appropriate combination of these properties.

The wide scale use of low power laser is known. One of such known therapeutic devices is the product manufactured by the Safe Laser Kft. commercially available under the trade name Safe Laser SL500 which is an infrared laser with nominal power of 500 mW that emits scattered infrared light at the wavelength of 808 nm. Its specification can be found at the internet address www.safelaser.hu. In the device Safe Laser 500 the laser light source is arranged in the middle of a tubular housing, and in front of it the head part of the device slightly expands along a cone, and in this part there is a beam expander and on the front portion thereof a large number of diffusers are arranged using respective small optical lenses, and in front of them only a transparent plastic protection lens is arranged. Because the laser light is emitted in the infrared range which is not visible, the laser is surrounded by four LED light sources emitting red light (in the wavelength of 630 nm) which are arranged at corner points of a square fitted in a circle surrounding the laser source, and these LEDs are operated together with the laser to indicated by their visible red light that the laser is switched on.

Although the mentioned device can be used for a high number of therapeutic purposes, several diseases or symptoms cannot be healed or relieved because the ill tissues and joints lie deeply under the body surface, and their geometrical sizes are substantially greater than the efficient area of the irradiation of the mentioned device in such depth. Theoretically there would be a possibility to increase the power of the laser light, but this has limitations, because owing to security reasons the use of soft laser light is not allowed if its power density at the body surface exceeds a threshold value. The aforementioned device of the type SL500 emits a radiation in an area with a diameter of about 30 mm which has a density close to the permitted threshold. The increase of power is therefore not a possible way for the treatment of deep tissues of large size.

In living tissues the power density and distribution of the laser light emitted by the device SL500 rapidly decreases with the depth and the diameter of the spot associated with the given power density also decreases.

The question has emerged to arrange several ones of such light sources beside each other, but the head diameter of the devices being around 35mm impose a limit to the smallest distance between such devices, but if one takes into account that in deeper regions the efficient spot size is only a fraction of the initial diameter, it is obtained that the use of several SL500 devices in a side-by-side arrangement could realize only a very inhomogeneous treatment, and during the use of several devices the penetration depth would not increase compared to the depth with a single device.

US 2008/125835 A1 discloses a low power therapy laser device comprising a housing which has a wider head portion that has a hollow cavity open at the front, and laser diodes configured to project a beam through a lens which may be,in the form of an expander lens configured to project a focused beam from laser diode

The task of the invention is to provide a low power laser device which satisfies the power density conditions at the skin surface whereas its penetration into deeper layers and the irradiated spot area in such depth is substantially greater than in case of using a single device.

For solving this task I have provided an improved low power therapy laser device that is structured as defined in the attached claims.

The solution according to the invention will now be described in connection with preferred embodiments thereof, wherein reference will be made to the accompanying drawings. In the drawing:
Fig. 1 is the simplified elevation sectional view of the device according to the invention;
Fig. 2 is the view of the device shown in Fig. 1 sown from axial direction;
Fig. 3 is the sketch of the head portion of the device together with the radiation intensity curve

in the depth of body tissues that also shows the curve of the device type SL500; and

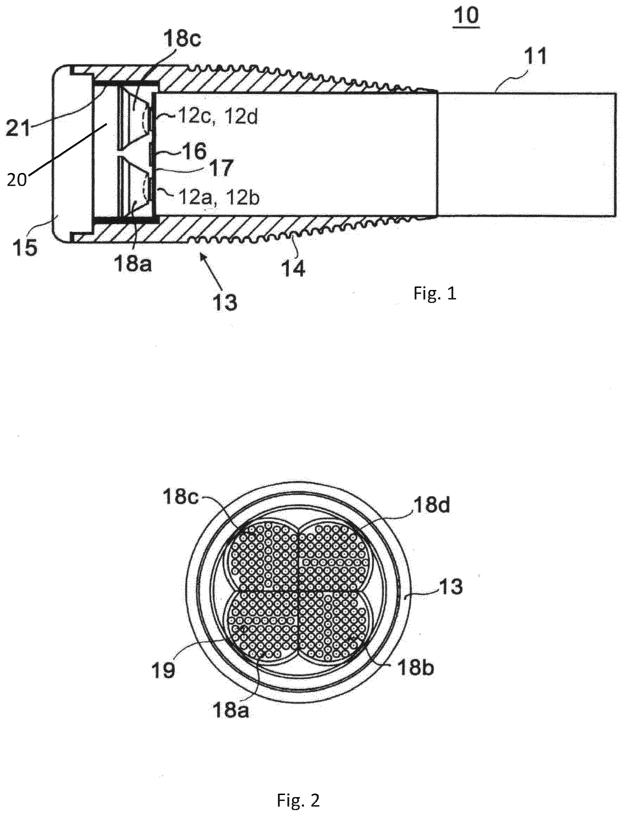

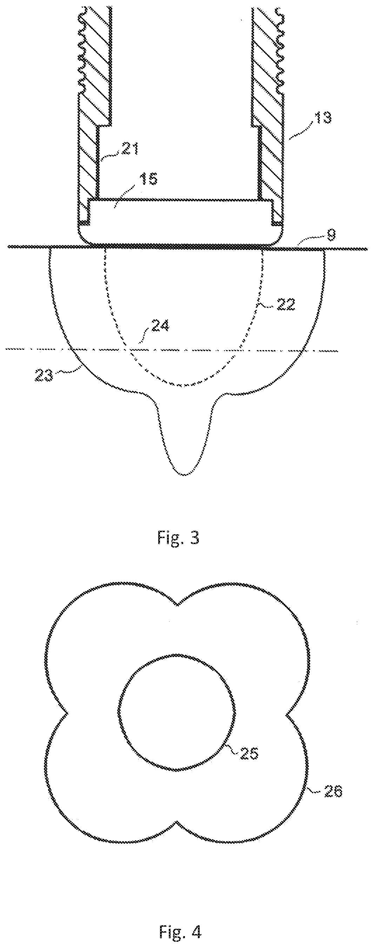

Fig. 1 shows the cross section of the curve of Fig. 3 measured in a depth in which the device type SL500 is still efficient.

The device 10 shown in Fig. 1 has a cylindrical housing 11 and in the interior thereof four batteries (not shown in the drawing) operate four lasers 12a, 12b, 12c and 12d arranged in the corners of a square form. In the longitudinal sectional view of Fig. 1 the lasers 12a and 12c are visible and the lasers 12b and 12d are covered. The chosen lasers have identical type and belong to the group of light power lasers, their power falls in the range between 40 and 500 mW. In the exemplary case the power is just 500mW, and the wavelength of the emitted light is between 600nm and 1500nm, preferably between 780 and 950 nm. In the exemplary case the wavelength of the lasers 12a, 12b, 12c and 12d is 808 nm, and they emit their light along respective straight slots. Although several other laser types are known, the chosen slot-radiation type lasers have high reliability and provide operation during a long lifetime. The direction of the slots of the four lasers 12a, 12b, 12c and 12d are such that in clockwise direction the subsequent slots close an angle of 90° with the direction of the previous one. The housing 11 has a head portion 13 with conical profile and it comprises cooling ribs 14 at its outer surface, and the head portion 13 extends in forward direction in a given distance over relative to the forward faces of the lasers 12a, 12b, 12c and 12d. The head portion 13 has a hollow interior closed at the front by a plastic closing element 15.

In the central line between the lasers 12a, 12b, 12c and 12d a LED light source 16 is arranged which has the same role as the LED light sources used in the known device type SL500, i.e. its visible light indicates that the lasers 12a, 12b, 12c and 12d are all operating.

The lasers 12a, 12b, 12c and 12d and the LED light source 16 are held by a support structure not shown in the drawing that has a face plate 17 arranged normal to the axis of the housing 11 and it is located slightly rearward compared to the front of the light sources and therefore it comprises openings for letting the body of the sources extend therethrough. The front surface of the face plate 17 is a light reflecting mirror surface. In front of the respective lasers 12a, 12b, 12c and 12d beam expanders 18a, 18b, 18c and 18d are arranged that all have special shapes which are fitted in a groove provided in the hollow interior of the head portion 13 and they are abutting and are connected to each other along respective planar surfaces. The front view of the beam expanders 18a, 18b, 18c and 18d can be observed in Fig. 2, and out of these beam expanders in Fig. 1 only the beam expanders 18a and 18c can be seen. The beam expanders 18a, 18b, 18c and 18d all have concave rear surfaces illustrated in Fig.1 by the dashed lines. Although the beam expanders 18a, 18b, 18c and 18d are interconnected along planar surfaces, their design is basically conical ad their task lies in expanding the incident laser beams broken by the concave lens. The beam expanders 18a, 18b, 18c and 18d are made by optically clean plastic material that retains the polarization of the light, e.g. of plexi material. On the frontal surfaces of the beam expanders 18a, 18b, 18c and 18d respective pluralities of diverting lenses 19 are provided which are made of small swellings and arranged along a regular raster that has the task of the more efficient diverting of the trough-passing light.

The head portion 13 has a cylindrical cavity 20 that holds the beam expanders 18a, 18b, 18c and 18d that extend in forward direction over the front faces of the beam expanders 18a, 18b, 18c and 18d and the cavity 20 lasts in rearward direction till the face plate 17. It is also preferred if the cylindrical surface 21 of the cavity 20 is provided with a light reflecting mirror coating because the laser beams arrive not only from the light slots of the lasers 12a, 12b, 12c and 12d to the concave surface of the beam expanders 18a, 18b, 18c and 18d but also by means of multiple reflections from the mirror surface of the face plate 17 and from the mirroring inner surface of the cavity 20. Owing to such an arrangement the light leaving the closing element 15 will become a uniformly scattered light.

Reference is made now to Figs. 3 and 4 in which the results of intensity distribution measurements were show in case of the device 10 according to the invention and of a known device of the type SL500 with a power of 500 mW in case of measuring meat that has absorption similar to human tissues. Fig. 3 shows only schematically the front part of the head portion 13 of the device 10 according to the invention, wherein during the measurements the face plane of the closing element 15 contacted the skin surface 9 on the meat under examination. During the comparative reference measurements the front surface of the device SL 500 was also placed to the same surface. The dashed line 22 relates to the distribution with the reference device at an intensity value which still has a certain therapeutic effect. The distribution of the intensity measured by the device 10 according to the invention is shown by the curve 23 drawn by the unbroken line.

The intensity distribution parallel to the skin surface has been examined at a given depth as shown in Fig. 3 by the dash-dotted line 24. The result of this examination is shown by the distribution curves of Fig. 4, in which the inner circular area 25 shows the distribution obtained by the reference device, and the distribution area 26 which is similar to a quatrefoil that surrounds the area 25 is obtained by the device 10 according to the invention. It should be noted that within the given area 25 the intensity was not the same at each position, but in all points it was greater than a threshold value. Both the distribution along an area of given depth and according to the function of the depth are very surprising, because one would have expected that the arrangement with four sources would have provided four times of the distribution obtained by the reference device, i.e. only the size of the area would have changed and the penetration depth would have remained the same, whereas the respective distributions would have appeared separately and between them sharp minimums would have appeared. It should also be noted that the intensity on the skin surface 9 was always under the permitted limit value.

In contrast to such expectations the results obtained by the device 10 have demonstrated substantial increase both in depth and in the size of the area. The reason of such results may lie in the fact that the laser light leaving the face plane of the closing element 15 is scattered to such an extent that in the deeper areas the components coming from the different laser sources get added to each other because a non-negligible portion of the energy of each laser is scattered under the adjacent areas and vice versa, and as a result of such a phenomenon a common area 26 is obtained with the shown shape of a quatrefoil. This large unbroken area has a high therapeutic significance because during the irradiation no spots will be found within the treated area, which have intensity minimums insufficient for the treatment. The increase of the depth which is efficient from the point of view of therapy has also an outstanding significance. If we take a look on the lower peak of the dashed line 22 in Fig. 3 which shows the points which are still good from the point of view of a therapy, then at the peak this efficient intensity is available in only a single point, whereby the reference device is practically useless in such a depth. In contrast thereto if the device 10 according to the invention is used, then in the same depth the size of the efficiently irradiated area is about as large as the diameter of the head portion 13.

By using the device 10 according to the invention comparatively few experimental results are available, but these are very positive. Significant improvement has been experienced primarily in case of treating articular and cartilage problems. Symptoms at the knee joints and in case of knee arthrosis and spots injuries have reacted surprisingly well to the therapy by the device 10. In the foregoing an embodiment of the device 10 according to the invention using four pieces of lasers with a power of 500 mW each has been shown. It is thought that such a size is already appropriate for treating sufficiently large areas, and can still be conveniently held in hand and uses a battery (i.e. independent from the electric grid), and its cooling can be solved without the need of a separate fan or other cooling unit. Of course, the number of the lasers can be increased or decreased e.g. to three, but if the number of the lasers is increased over four, then the related problems of cooling and proper power supply and electric safety should also be solved.

In the example four separate beam expanders 18a, 18b, 18c and 18d were used, however these can also be realized by a single common plastic body, therefore the invention is not limited to the embodiments shown only by way of examples.

## Claims

1. Improved low power therapy laser device (10) comprising a housing (11) which has a wider head portion (13) that has a hollow cavity (20), a laser light source arranged in the housing (10), a beam expander arranged in the beam path of the laser and also diverting lenses (19), and the hollow cavity (20) of the head portion (13) is closed at the front by a transparent closing element (15), **characterized in that** in the cavity (20) of the head portion (13) in an even circular distribution at least three lasers (12a, 12b, 12c 12d) are provided, and in front of them respective beam expanders (18a, 18b, 18c and 18d) are arranged, the beam expanders (18a, 18b, 18c and 18d) contact and support each other along respective surfaces, and the diverting lenses (19) are provided on the frontal surfaces of the beam expanders (18a, 18b, 18c and 18d) and are made as small swellings, said diverting lenses (19) are arranged along a raster and have the task of providing a more efficient diverting of the trough-passing light, and the laser light leaving the face plane of the closing element (15) is distributed throughout the whole closing element (15) and scattered to such an extent that in the deeper areas the components coming from the different laser sources get added to each other.

2. The device as claimed in claim 1, wherein the lasers (12a, 12b, 12c, 12d) are inserted in respective openings of a face plate (17) closing the rear end of the cavity (20) of the head portion (13), and the face plate (17) has a light reflective front surface facing towards the interior of the cavity (20).

3. The device as claimed in claims 1 or 2, wherein the cavity (20) of the head portion (13) has a cylindrical shape and in forward direction it slightly extends over the beam expanders (18a, 18b, 18c, 18d) and has a light reflecting inner surface.

4. The device as claimed in any of claims 1 to 3, wherein the lasers (12a, 12b, 12c, 12d) emit light falling in the wavelength range of 600 to 1500 nm, and their respective powers is higher than 40mW.

5. The device as claimed in claim 4, wherein among the lasers (12a, 12b, 12c, 12d) in a central location a LED light source (16) emitting visible light is arranged which is switched on and off together with the lasers (12a, 12b, 12c, 12d).

6. The device as claimed in claims 4 or 5, wherein four lasers (12a, 12b, 12c and 12d) are used and they are arranged in the corner points of a square shape.

7. The device as claimed in claim 6, wherein the lasers (12a, 12b, 12c and 12d) emit light along respective linear slots.

8. The device as claimed in claim 6 or 7, wherein the beam expanders (18a, 18b, 18c, 18d) meet each other or being built together along respective planes parallel to the sides of said square and the centre of which falls in the axis of the cavity (20).

9. The device as claimed in claim 8, wherein the beam expanders (18a, 18b, 18c and 18d) are made of a single transparent body.

10. The device as claimed in any of the claims 1 to 9, wherein the head portion (13) of the housing (11) has a conical profile and comprises cooling ribs (14) at its outer surface.

11. The device as claimed in any of the claims 1 to 10, wherein the lasers (12a, 12b, 12c, 12d) have power of 500 mW each.

12. The device as claimed in claim 11, wherein the intensity of the laser light leaving the closing element (15) is close to the threshold permitted for reaching the skin surface.

## Patentansprüche

1. Verbessertes Niedrigleistungs-Therapielasergerät (10) umfassend ein Gehäuse (11), das einen breiteren Kopfabschnitt (13) mit einem Hohlraum (20) aufweist, eine im Gehäuse (10) angeordnete Laserlichtquelle, einen im Strahlengang des Lasers angeordneten Strahlaufweiter sowie Umlenklinsen (19), wobei der Hohlraum (20) des Kopfabschnitts (13) nach vorne durch ein transparentes Verschlusselement (15) verschlossen ist, **dadurch gekennzeichnet, dass** in dem Hohlraum (20) des Kopfabschnitts (13) in gleichmäßiger kreisförmiger Verteilung mindestens drei Laser (12a, 12b, 12c, 12d) vorgesehen sind und davor jeweils Strahlaufweiter (18a, 18b, 18c und 18d) angeordnet sind, wobei die Strahlaufweiter (18a, 18b, 18c und 18d) einander entlang jeweiliger Oberflächen berühren und stützen, und wobei die Umlenklinsen (19) an den Stirnflächen der Strahlaufweiter (18a, 18b, 18c und 18d) vorgesehen und als kleine Wölbungen ausgebildet sind, wobei die Umlenklinsen (19) längs eines Rasters angeordnet sind und die Aufgabe haben, für eine effizientere Umlenkung des durchtretenden Lichts zu sorgen, und das die Stirnfläche des Verschlusselements (15) verlassende Laserlicht über das gesamte Verschlusselement verteilt (15) und so stark gestreut wird, dass sich in den tieferen Bereichen die von den verschiedenen Laserquellen stammenden Komponenten addieren.

2. Vorrichtung nach Anspruch 1, wobei die Laser (12a, 12b, 12c, 12d) in entsprechende Öffnungen einer Frontplatte (17), die das hintere Ende des Hohlraums (20) des Kopfabschnitts (13) verschließt, eingesetzt sind, und wobei die Frontplatte (17) eine lichtreflektierende Vorderfläche aufweist, die dem Inneren des Hohlraums (20) zugewandt ist.

3. Vorrichtung nach Anspruch 1 oder 2, wobei der Hohlraum (20) des Kopfabschnitts (13) eine zylindrische Form aufweist und sich in Vorwärtsrichtung leicht über die Strahlaufweiter (18a, 18b, 18c, 18d) erstreckt, und eine lichtreflektierende Innenfläche aufweist.

4. Vorrichtung nach einem der Ansprüche 1 bis 3, wobei die Laser (12a, 12b, 12c, 12d) Licht, das in den Wellenlängenbereich von 600 bis 1500 nm fällt, emittieren und ihre jeweiligen Leistungen größer als 40 mW sind.

5. Vorrichtung nach Anspruch 4, **dadurch gekennzeichnet, dass** zwischen den Lasern (12a, 12b, 12c, 12d) an zentraler Stelle eine sichtbares Licht emittierende LED-Lichtquelle (16) angeordnet ist, die zusammen mit den Lasern (12a, 12b, 12c, 12d) ein- und ausgeschaltet wird.

6. Vorrichtung nach Anspruch 4 oder 5, **dadurch gekennzeichnet, dass** vier Laser (12a, 12b, 12c und 12d) verwendet werden und diese in den Eckpunkten einer quadratischen Form angeordnet sind.

7. Vorrichtung nach Anspruch 6, wobei die Laser (12a, 12b, 12c und 12d) Licht entlang jeweiliger linearer Schlitze emittieren.

8. Vorrichtung nach Anspruch 6 oder 7, wobei die Strahlaufweiter (18a, 18b, 18c, 18d) einander treffen oder entlang jeweiliger Ebenen, die parallel zu den Seiten des Quadrats sind und deren Mitte in die Achse des Hohlraums (20) fällt, zusammengebaut sind.

9. Vorrichtung nach Anspruch 8, **dadurch gekennzeichnet, dass** die Strahlaufweiter (18a, 18b, 18c und 18d) aus einem einzigen transparenten Körper bestehen.

10. Vorrichtung nach einem der Ansprüche 1 bis 9, **dadurch gekennzeichnet, dass** der Kopfabschnitt (13) des Gehäuses (11) ein konisches Profil aufweist und an seiner Außenfläche Kühlrippen (14) aufweist.

11. Vorrichtung nach einem der Ansprüche 1 bis 10, wobei die Laser (12a, 12b, 12c, 12d) jeweils eine Leistung von 500 mW aufweisen.

12. Vorrichtung nach Anspruch 11, wobei die Intensität des das Verschlusselement (15) verlassenden Laserlichts nahe der zum Auftreffen auf die Hautoberfläche zulässigen Schwelle liegt.

## Revendications

1. Dispositif laser de thérapie à faible puissance (10) amélioré comprenant un boîtier (11) qui a une partie de tête plus large (13) qui a une cavité creuse (20), une source de lumière laser disposée dans le boîtier (10), un élargisseur de faisceau disposé dans le trajet de faisceau du laser et également des lentilles de déviation (19), et la cavité creuse (20) de la partie de tête (13) est fermée à l'avant par un élément de fermeture transparent (15), **caractérisé en ce que** dans la cavité (20) de la partie de tête (13) sont fournis, selon une répartition circulaire régulière, au moins trois lasers (12a, 12b, 12c, 12d), devant lesquels sont disposés des élargisseurs de faisceau (18a, 18b, 18c et 18d) respectifs, les élargisseurs de faisceau (18a, 18b, 18c et 18d) sont en contact et se supportent mutuellement le long de surfaces respectives, et les lentilles de déviation (19) sont fournies sur les surfaces antérieures des élargisseurs de faisceau (18a, 18b, 18c et 18d) et sont réalisées comme de petits renflements, lesdites lentilles de déviation (19) sont disposées le long d'une trame et ont pour tâche de fournir une déviation plus efficace de la lumière passant en auge, et la lumière laser quittant le plan frontal de l'élément de fermeture (15) est répartie sur l'ensemble de l'élément de fermeture (15) et dispersée de telle sorte que, dans les zones plus profondes, les composants provenant des différentes sources laser s'ajoutent les uns aux autres.

2. Dispositif tel que revendiqué dans la revendication 1, dans lequel les lasers (12a, 12b, 12c, 12d) sont insérés dans des ouvertures respectives d'une plaque frontale (17) fermant l'extrémité arrière de la cavité (20) de la partie de tête (13), et la plaque frontale (17) a une surface avant réfléchissant la lumière tournée vers l'intérieur de la cavité (20).

3. Dispositif tel que revendiqué dans les revendications 1 ou 2, dans lequel la cavité (20) de la partie de tête (13) a une forme cylindrique et, dans la direction avant, elle s'étend légèrement au-dessus des élargisseurs de faisceau (18a, 18b, 18c, 18d) et a une surface interne réfléchissant la lumière.

4. Dispositif tel que revendiqué dans l'une quelconque des revendications 1 à 3, dans lequel les lasers (12a, 12b, 12c, 12d) émettent une lumière tombant dans la gamme de longueurs d'onde de 600 à 1500 nm, et leurs puissances respectives sont supérieures à 40 mW.

5. Dispositif tel que revendiqué dans la revendication 4, dans lequel parmi les lasers (12a, 12b, 12c, 12d), à un emplacement central, est disposée une source de lumière LED (16) émettant de la lumière visible qui est allumée et éteinte en même temps que les lasers (12a, 12b, 12c, 12d).

6. Dispositif tel que revendiqué dans les revendications 4 ou 5, dans lequel quatre lasers (12a, 12b, 12c et 12d) sont utilisés et sont disposés dans les points d'angle d'une forme carrée.

7. Dispositif tel que revendiqué dans la revendication 6, dans lequel les lasers (12a, 12b, 12c et 12d) émettent de la lumière le long de fentes linéaires respectives.

8. Dispositif tel que revendiqué dans la revendication 6 ou 7, dans lequel les élargisseurs de faisceau (18a, 18b, 18c, 18d) se rencontrent ou sont construits ensemble le long de plans respectifs parallèles aux côtés dudit carré et dont le centre tombe dans l'axe de la cavité (20).

9. Dispositif tel que revendiqué dans la revendication 8, dans lequel les élargisseurs de faisceau (18a, 18b, 18c et 18d) sont constitués d'un seul corps transparent.

10. Dispositif tel que revendiqué dans l'une quelconque des revendications 1 à 9, dans lequel la partie de tête (13) du boîtier (11) a un profil conique et comprend des nervures de refroidissement (14) sur sa surface extérieure.

11. Dispositif tel que revendiqué dans l'une quelconque des revendications 1 à 10, dans lequel les lasers (12a, 12b, 12c, 12d) ont chacun une puissance de 500 mW.

12. Dispositif tel que revendiqué dans la revendication 11, dans lequel l'intensité de la lumière laser quittant l'élément de fermeture (15) est proche du seuil autorisé pour atteindre la surface de la peau.
